Europäisches Patentamt

(19) European Patent Office

Office européen des brevets

(11) Publication number: **0 138 282**
**B1**

(12) **EUROPEAN PATENT SPECIFICATION**

(45) Date of publication of patent specification: **30.09.87**

(51) Int. Cl.⁴: **C 07 D 499/00**

(21) Application number: **84201514.1**

(22) Date of filing: **18.10.84**

(54) Process for the dehalogenation of 6,6-dibromopenicillanic acid 1,1-dioxide.

(30) Priority: **18.10.83 EP 83201500**

(43) Date of publication of application:
**24.04.85 Bulletin 85/17**

(45) Publication of the grant of the patent:
**30.09.87 Bulletin 87/40**

(84) Designated Contracting States:
**AT BE CH DE FR GB IT LI LU NL SE**

(56) References cited:
**EP-A-0 061 315**
**EP-A-0 092 286**
**GB-A-2 045 755**

(73) Proprietor: **Gist - Brocades N.V.**
**Wateringseweg 1 P.O. Box 1**
**NL-2600 MA Delft (NL)**

(72) Inventor: **Kapur, Jagdish Chander**
**Roland Holstlaan 799**
**NL-2624 KB Delft (NL)**
Inventor: **Fasel, Herman Pieter**
**Van der Haertstraat 19**
**NL-2613 XZ Delft (NL)**

(74) Representative: **Huygens, Arthur Victor, Dr. et al**
**c/o Gist-Brocades N.V. Patent & Trademarks Department Wateringseweg 1 PO Box 1 NL-2600 MA Delft (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 138 282

**Description**

The invention relates to a new process for the preparation of penicillanic acid 1,1-dioxide by dehalogenation of 6,6-dibromopenicillanic acid 1,1-dioxide.

The presumed association between the resistance of certain bacteria to bêta-lactum antibiotics and the capability of these bacteria to produce and secrete beta-lactamases has led to an intensive search for bêta-lactamase inhibitors.

It is known from Dutch patent application 7806126 that penicillanic acid 1,1-dioxide and salts and esters thereof, have useful pharmcological properties, for example as effective inhibitors of several types of bêta-lactamases present in various kinds of bacteria. In the beforementioned Dutch application a process is described for the preparation of penicillanic acid 1,1-dioxide and salts and esters thereof by oxidation of penicillanic acid.

Another process for the preparation of penicillanic acid, 1,1-dioxide is described in Dutch patent application 8001285. In this application penicillanic acid 1,1-dioxide is prepared by diazotisation-bromination of 6-amino-penicillanic acid followed by oxidation of the formed 6,6-dibromopenicillanic acid into 6,6-dibromopenicillanic acid 1,1-dioxide and dehalogenation of the latter compound.

The preferred dehalogenation reaction of 6,6-dibromopenicillanic acid 1,1-dioxide described in the before-mentioned application is the reduction with hydrogen in the presence of a palladium catalyst. This process has the disadvantage that it uses the highly flammable and explosive hydrogen gas and that special equipment has to be used to carry out the reaction under a pressure of 2 to 5 atmospheres.

Two other dehalogenation methods are described in the before-mentioned application. These reactions, i.e. the reduction with zinc in a phosphate buffer or in acetic acid and the reduction with tributyl tinhydride, however, give low yields of often impure product.

It has now surprisingly been found that penicillanic acid 1,1-dioxide of high purity can be prepared in an excellent yield by reduction of 6,6-dibromopenicillanic acid 1,1-dioxide with magnesium in association with an acid. The application of magnesium metal as an excellent dehalogenation reagent under mild conditions is hitherto unknown in the literature. The present invention, therefore, relates to a process for the preparation of penicillanic acid 1,1-dioxide by dehalogenation of 6,6-dibromopenicillanic acid characterized in that the reaction is effected with magnesium in association with an acid.

This result is even more surprising because experiments to reduce in the same way esters of 6,6-dibromo-penicillanic acid, for instance the methyl and pivaloyl ester did not succeed.

When using the process of the present invention it is possible to prepare penicillanic acid 1,1-dioxide in a yield of 90%. In comparison with the process described in the beforementioned Dutch patent application 8001285 a relative improvement of the yield of penicillanic acid 1,1-dioxide of 50% is reached.

Furthermore it is not necessary any more to use the highly flammable and explosive hydrogen gas, and the reaction can be performed in the usual equipment without any additional requirements to carry out the reaction under high pressure.

It is another advantage of the present invention that penicillanic acid 1,1-dioxide of very high purity is obtained, thus avoiding an additional purification procedure. The purity of the product which is isolated directly from the reaction mixture amounts to at least 95%. This product is contaminated with less than 5 ppm of magnesium. In this respect, it is remarked that a product prepared by reduction with hydrogen in association with a palladium catalyst very often is contaminated with a comparatively high amount of palladium. Furthermore, the product has a white colour which is important for substances to be used for the preparation of pharmaceutical products.

In European patent application No. 83200542, publication No. 0092286, which is not prepublished, a process is described for the dehalogenation of 6-alpha-bromopenicillanic acid 1,1-dioxide and 6,6-dibromopenicillanic acid 1,1-dioxide by reduction with zinc in association with an acid having a pKa-value measured in water of less than 3.5 in a watercontaining medium. The present invention also shows improvements with respect to this process. The most important improvement of the new method of reduction with magnesium is that the product is completely colourless, while in the case of reduction with zinc the product has always a yellow colour, sometimes even a light brown colour. This is especially important when the compound is used for the preparation of a pharmaceutical preparation, for which it is always very important to contain as little contaminants, especially coloured contaminants, as possible. Other improvements are the slightly higher yields, the fact that the reaction can be performed at a somewhat lower temperature in the same time, and that the price of magnesium is lower than the price of the equivalent amount of zinc. Furthermore, the magnesium salts which are formed as byproducts of the new process will form a smaller contamination load for the environment than the zinc salts formed in the other process.

Examples of acids which can be used in the present invention are hydrochloric acid, hydrobromic acid, sulphuric acid, boric acid, perchloric acid, aryl sulphonic acids (e.g. p-tolylsulphonic acid) and sufficiently acidic alkanoic acids and alkanoic diacids. Preferably hydrochloric acid, hydrobromic acid and sulphuric acid are used, more preferably hydrochloric acid.

The reaction is preferably carried out at a pH of 2.5 to 7, more preferably 4 to 6.

The reaction is carried out in a mixture of a water-miscible or partly water-miscible inert organic

2

solvent and water. Suitable organic solvents are ethyl acetate, butyl acetate and acetonitrile. Preferably ethyl acetate is used.

The reaction is preferably carried out at a temperature between −10 and 25°C, more preferably between −3 and 10°C.

Preferably magnesium powder is used in the reaction.

The following non-limitative examples illustrate the present invention. General remarks:

1. a. The purity of the 6,6-dihalo- or 6-alpha-halopenicillanic acid 1,1-dioxide was determined by means of its 60 MHz NMR spectrum in acetone-$d_6$ using 2,6-dichloroacetophenone as the reference.

1. b. The purity of penicillanic acid 1,1-dioxide (PAS) was determined by means of HPLC analysis using a standard preparation whose purity has been established through its 360 MHz NMR spectrum in acetone-$d_6$ with 2,6-dichloroacetophenone as the reference.

2. Magnesium metal powder as used in the present invention was purchased from Riedal-De Haën AG.

Example 1

To a well-stirred solution of 6,6-dibromopenicillanic acid 1,1-dioxide (6.0 g; purity by 60 MHz NMR spectrum in acetone-$d_6$ using 2,6-dichloroacetophenone as the reference: 97.35%; 14.9 mmol) in ethyl acetate (150 ml) and water (35 ml) kept at −2° to 3°C was added portionwise magnesium powder (3.8 g), while maintaining the pH of the reaction at 3.5 with 4N hydrochloric acid. The contents were further stirred for 2 hours while maintaining the pH at 3.5 with 4N hydrochloric acid and the temperature at −2° to 3°C. Thereafter, the solid was filtered, washed with water and ethyl acetate. The combined filtrate was brought to pH 2.0 with 4N hydrochloric acid, whereupon the layers were separated. The aqueous layer was extracted with ethyl acetate (3×80 ml) after which the combined extracts were washed with brine (2×60 ml), dried over anhydrous magnesium sulfate, filtered and evaporated under reduced pressure to afford a white solid product which was taken up in n-hexane, filtered, evaporated and dried under reduced pressure to afford penicillanic acid 1,1-dioxide=3.125 g (purity by 360 MHz spectrum=96.3%), thus giving a yield of 87%.

Example 2

The reaction was carried out as described in Example 1, using 6,6-dibromopenicillanic acid 1,1-dioxide (6.0 g; purity by NMR 95.85%; 14.7 mmol) and Mg (powder, 4.6 g) at pH=2 (maintained with 4N HCl) and a reaction time of 5 hrs. Isolated yield of penicillanic acid 1,1-dioxide=3.092 g (purity by HPLC=75%), thus giving a yield of 68%.

Example 3

The reaction was carried out as described in Example 1, using 6,6-dibromopenicillanic acid 1,1-dioxide (6.0 g; purity by NMR=95.7%; 14.9 mmol) and Mg (powder, 2.44 g) at pH=4 (maintained with 4N HCl) and a reaction time of 4 hrs. Isolated yield of penicillanic acid 1,1-dioxide=3.117 g (purity by HPLC=96%), thus giving a yield of 87%.

Example 4

The reaction was carried out as described in Example 1, using 6,6-dibromopenicillanic acid 1,1-dioxide (6.0 g; purity by NMR=98.6%; 15.13 mmol) and Mg (powder, 2.13 g) at pH=5 (maintained with 4N HCl) and a reaction time of 3.5 hrs. Isolated yield of penicillanic acid 1,1-dioxide=3.221 g (purity by HPLC=95%), thus giving a yield of 87%.

Example 5

The reaction was carried out as described in Example, 1 using 6,6-dibromopenicillanic acid 1,1-dioxide (6.0 g; purity by NMR=96.5%; 14.8 mmol) and Mg (powder, 2.34 g) at pH=6 (maintained with 4N HCl) and a reaction time of 4.16 hrs. Isolated yield of penicillanic acid 1,1-dioxide=3.112 g (purity by HPLC=96%), thus giving a yield of 87%.

Example 6

The reaction was carried out as described in Example 1, using 6,6-dibromopenicillanic acid 1,1-dioxide (6.0 g; purity by NMR=96%; 14.7 mmol) and Mg (powder, 2.5 g) at a temperature of 5° to 10°C, and a reaction time of 2.5 hrs. Isolated yield of penicillanic acid 1,1-dioxide=3.045 g (purity by HPLC=94%), thus giving a yield of 83%.

Example 7

The reaction was carried out as described in Example 1, using 6,6-dibromopenicillanic acid 1,1-dioxide (6.0 g; purity by NMR=96%; 14.7 mmol) and Mg (powder, 2.44 g) at a temperature of 22°C and a reaction time of 2.45 hrs. Isolated yield of penicillanic acid 1,1-dioxide =2.894 g (purity by HPLC=95.5%), thus giving a yield of 80%.

Example 8

The reaction was carried out as described in Example 1, using 6,6-dibromopenicillanic acid 1,1-dioxide (6.0 g; purity by NMR=96%; 14.7 mmol) and Mg (powder 2.01 g) in butylacetate (instead of ethyl acetate) and a reaction time of 2 hrs. Isolated yield of penicillanic acid 1,1-dioxide=3.091 g (purity by HPLC=95.5%), thus giving a yield of 86%.

Example 9

The reaction was carried out as described in Example 1, using 6,6-dibromopenicillanic acid 1,1-dioxide (6.0 g; purity by NMR=96%; 14.7 mmol) and Mg (powder 4.3 g) in methyl acetate (instead of ethyl acetate) and a reaction time of 7.4 hrs. Isolated yield of penicillanic acid, 1,1-dioxide=2.545 g (purity by HPLC=94%), thus giving a yield of 70%.

Example 10

The reaction was carried out as described in Example 1, using 6,6-dibromopenicillanic acid 1,1-dioxide (6.09 g, purity by NMR=95.6%; 14.7 mmol) and Mg (powder, 3.8 g) in acetonitrile (instead of ethyl acetate) and a reaction time of 4 hrs. Isolated yield of penicillanic acid 1,1-dioxide=2.792 g (purity by HPLC=92%), thus giving a yield of 75%.

Example 11

The reaction was carried out as described in Example 1, using 6,6-dibromopenicillanic acid 1,1-dioxide (6.0 g; purity by NMR=96.25%; 14.8 mmol), Mg (powder, 4.55 g) and 4N $H_2SO_4$ (instead of 4N HCl) and a reaction time of 12 hrs. Isolated yield of penicillanic acid 1,1-dioxide=2.985 g (purity by HPLC=69.5%), thus giving a yield of 60%.

Example 12

The reaction was carried out as described in Example 1, using 6,6-dibromopenicillanic acid (6.0 g; purity by NMR=95.1%; 14.6 mmol), Mg (powder, 2.7 g) and 4N HBr (instead of 4N HCl) and a reaction time of 4 hrs. Isolated yield of penicillanic acid 1,1-dioxide=3.034 g (purity by HPLC=94%), thus giving a yield of 84%.

Example 13

The reaction was carried out as described in Example 1, using 6,6-dibromopenicillanic acid 1,1-dioxide (6.0 g; purity by NMR=94.65%; 14.5 mmol), Mg (powder, 2.48 g) and 4N $HCO_4$ (instead of 4N HCl) and a reaction time of 4 hrs. Isolated yield of penicillanic acid 1,1-dioxide=2.44 g (purity by HPLC=93%), thus giving a yield of 67%.

Example 14

The reaction was carried out as described in Example 1, using 6,6-dibromopenicillanic acid 1,1-dioxide (6.0 g; purity by NMR=96%; 14.7 mmol), Mg (3.8 g) and boric acid (solid) (instead of 4N HCl) at pH=7 (instead of 3.5) and a reaction time of 4 hrs. Isolated yield of penicillanic acid 1,1-dioxide=2.256 g (purity by HPLC=93%), thus giving a yield of 65.7%.

**Claims**

1. Process for the preparation of penicillanic acid 1,1-dioxide by dehalogenation of 6,6-dibromopenicillanic acid 1,1-dioxide characterized in that the reaction is effected with magnesium in association with an acid, in a mixture of a water-miscible or partly water-miscible inert organic solvent and water.

2. Process according to Claim 1, characterized in that the acid is hydrochloric acid, hydrobromic acid or sulphuric acid, preferably hydrochloric acid.

3. Process according to Claims 1—2, characterized in that the pH at which the debromination is effected is 2.5 to 7, preferably 4 to 6.

4. Process according to Claims 1—3, characterized in that the reaction is performed at a temperature between −10 and 25°C, preferably between −3° and 10°C.

5. Process according to Claims 1—4, characterized in that the reaction is carried out in ethyl acetate.

**Patentansprüche**

1. Verfahren zur Herstellung von Penicillansäure-1,1-dioxid durch Dehalogenierung von 6,6-Dibrompenicillansäure-1,1-dioxid, dadurch gekennzeichnet, dass die Reaktion mit Magnesium in Kombination mit einer Säure in einem Gemisch aus einem mit Wasser mischbaren oder teilweise mit Wasser mischbaren inerten organischen Lösungsmittel und Wasser ausgeführt wird.

2. Verfahren nach Anspruch 1, dadurch gekennzeichnet, dass die Säure Salzsäure, Bromwasserstoffsäure oder Schwefelsäure, vorzugsweise Salzsäure, ist.

3. Verfahren nach den Ansprüchen 1 bis 2, dadurch gekennzeichnet, dass der pH-Wert, bei dem die Debromierung ausgeführt wird, 2,5 bis 7, vorzugsweise 4 bis 6, beträgt.

4. Verfahren nach den Ansprüchen 1 bis 3, dadurch gekennzeichnet, dass die Reaktion bei einer Temperatur zwischen −10 und 25°C, vorzugsweise zwischen −3 und 10°C, ausgeführt wird.

5. Verfahren nach den Ansprüchen 1 bis 4, dadurch gekennzeichnet, dass die Reaktion in Ethylacetat ausgeführt wird.

**Revendications**

1. Procédé de préparation du 1,1-dioxyde d'acide pénicillanique par déshalogénation du 1,1-dioxyde d'acide 6,6-dibromopénicillanique, caractérisé en ce que la réaction est effectuée au moyen de magnésium en association avec un acide, dans un mélange d'eau et d'un solvant organique inerte miscible à l'eau ou partiellement miscible à l'eau.

2. Procédé suivant la revendication 1, caractérisé en ce que l'acide est l'acide chlorhydrique, l'acide bromhydrique, ou l'acide sulfurique, mais de préférence l'acide chlorhydrique.

3. Procédé suivant les revendications 1 et 2, caractérisé en ce que le pH auquel la débromation est exécutée est de 2,5 à 7 et de préférence de 4 à 6.

4. Procédé suivant les revendications 1 à 3, caractérisé en ce que la réaction est exécutée à une température entre −10 et 25°C et de préférence entre −3 et 10°C.

5. Procédé suivant les revendications 1 à 4, caractérisé en ce que la réaction est exécutée dans l'acétate d'éthyle.